# EUROPÄISCHE PATENTSCHRIFT

(11) **EP 2 927 308 B1**
(45) Veröffentlichungstag und Bekanntmachung des Hinweises auf die Patenterteilung: **18.03.2020**
(21) Anmeldenummer: 15000919.9
(22) Anmeldetag: 27.07.2007
(51) Int. Cl.: C12M 1/16, C12M 3/00, C12M 1/107, C12M 1/00

(54) **BIOGASANLAGE UND VERFAHREN ZUR ERZEUGUNG VON BIOGAS AUS STROH**
BIOGAS SYSTEM AND METHOD FOR GENERATING BIOGAS FROM STRAW
INSTALLATION DE BIOGAZ ET PROCÉDÉ DE PRODUCTION DE BIOGAZ À PARTIR DE PAILLE

(30) Priorität: 27.06.2007 DE 102007029700
(43) Veröffentlichungstag der Anmeldung: 07.10.2015
(62) Teilanmeldung aus: 07786393.4
(73) Patentinhaber: Meissner, Jan A., 8700 Küsnacht (CH)
(72) Erfinder: Feldmann, Michael, 35037 Marburg (DE)
(74) Vertreter: Schüssler, Andrea

(56) Entgegenhaltungen:
- EP-A- 0 286 100
- EP-A- 0 934 998
- WO-A1-2004/016797
- DE-A1- 2 730 671
- DE-A1- 4 308 920
- DE-A1- 10 021 383
- US-A1- 2002 102 673
- US-A1- 2002 192 774
- US-A1- 2003 176 669
- US-A1- 2006 024 801
- US-A1- 2006 275 895

## Beschreibung

Die vorliegende Erfindung betrifft eine Biogasanlage zur Biogaserzeugung nach dem Oberbegriff von Anspruch 1 und ein Verfahren zur Erzeugung von Biogas aus Stroh.

Gegenwärtig findet Stroh in Biogasanlagen praktisch keine Anwendung als Gärsubstrat; in Biogasanlagen gelangt es lediglich indirekt in kleinen Mengen als im Festmist enthaltene Einstreu. Es besteht nämlich aus Gründen, die im Folgenden erläutert werden, ein technisches Vorurteil gegenüber der Verwendung von Stroh als Gärsubstrat.

Gegenwärtig sind in Deutschland über 3.500 Naßfermentationsanlagen im Einsatz, denen lediglich rund 20 Anlagen zur Feststoffvergärung (Trockenvergärung) von nachwachsenden Rohstoffen gegenüberstehen. Bei Naßfermentationsanlagen kommt eine Verwendung von Stroh schon deshalb nicht in Betracht, weil die nasse Gärsubstanz mit Paddelrädern bzw. Propellern umgerührt werden muß und sich das Stroh dabei um die Paddel bzw. Propeller wickeln würde. Deshalb wird in Naßfermentationsanlagen auch kaum strohenthaltender Festmist verwendet, sondern vornehmlich Gülle. Wenn man das Stroh häckseln würde, um dieses mechanische Problem zu umgehen, schwimmt das gehäckselte Stroh auf und mischt sich daher mit dem nassen Gärsubstrat nicht. Außerdem verstopft es dabei in der Regel die Ab- bzw. Überläufe der Nassfermenter. Schon aus diesen Gründen kommt Stroh als Gärsubstrat für Naßfermentationsanlagen gegenwärtig nicht in Betracht.

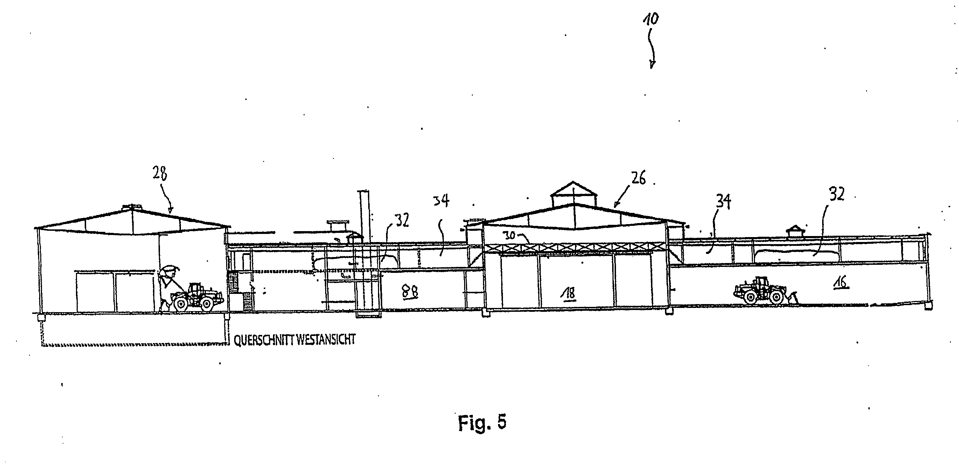

Bestehende Biomasse-Kraftwerke bzw. Biogasanlagen zur Biogaserzeugung nach dem Feststoff-Vergärungsverfahren sind typischerweise kleine landwirtschaftliche Anlagen mit zwei bis sechs Fermentern. Gemäß dem erst kürzlich erschienenen Endbericht "Monitoring zur Wirkung des novellierten Erneuerbare-Energien-Gesetzes (EEG) auf die Entwicklung der Stromerzeugung aus Biomasse", der im Auftrag des Bundesministeriums für Umwelt, Naturschutz und Reaktorsicherheit (BMU) erstellt wurde, hat die Trockenvergärung noch nicht den Stand der Marktreife erreicht. Die in Betrieb befindlichen Anlagen werden dort vielmehr als Demonstrationsanlagen angesehen (siehe Seite 52, Abb. 5-2 des genannten Endberichts).

Prinzipiell wäre es zwar möglich, in solchen Feststoffvergärungsanlagen Stroh als Gärsubstrat zu verwenden. Dies wird jedoch gegenwärtig nicht getan, weil gemeinhin angenommen wird, daß der Gasertrag von Stroh zu gering sei.

Anstelle von Stroh werden in bekannten Feststoffvergärungsverfahren statt dessen herkömmlicherweise vorwiegend Bioabfälle sowie Rinder- und Schweinefestmist und Hühnertrockenkot verwendet. Außerdem werden hauptsächlich wenig lignifizierte NawaRos, wie beispielsweise frisch geerntetes Gras, Silagen aus Gras, Getreide-Ganzpflanzenschnitt und Mais-Ganzpflanzenschnitt sowie Heu, Kartoffeln und Rübenhackschnitzel eingesetzt.

Da die gegenwärtig bekannten Biogasanlagen mit Feststoffvergärung typischerweise im ländlichen Raum, beispielsweise auf einem Bauernhof stehen und nur einen verhältnismäßig kleinen Durchsatz haben, wird der Bedarf an Gärmasse üblicherweise aus dem eigenen Bestand des Landwirtes und möglicherweise der Nachbarbetriebe gedeckt. Für größere Feststoffvergärungsanlagen als bisher üblich stellt die Beschaffung geeigneter Gärsubstrate in ausreichender Menge und zu wirtschaftlichen Bedingungen jedoch ein Problem dar.

WO2004/016797 A1 beschreibt ein Verfahren und eine Anlage zur Erzeugung von Biogas aus organischem Material. Diese besteht im Wesentlichen aus einer Standard- bzw. Getreidekornmühle (ggf. auch nur aus einer einfachen Mühle für die Zerreibung von Grünmassepellets), einem Anmischtank mit einem Rührwerk, einem Fermentationsreaktor mit einem Rührwerk, Pumpen und einer Steuereinheit. Als Einsatzstoff kann jede Art von Grünmasse dienen sowie Schlachtabfälle und Gülle. Ohne auf die unterschiedlichen spezifischen Eigenschaften der Einsatzstoffe einzugehen, versteht diese Anmeldung unter Grünmasse alle Pflanzen, die zur Erzeugung der Pflanzenmasse den natürlichen Prozess der Photosynthese nutzen wie z.B. Silagen, Stroh, Getreide, Spreu, Raps, Zuckerrüben, Steckrüben, Mais, Sonnenblumen, Kohl, Kartoffeln, Molasse, Erbsen, Bohnen, Linsen, Flachs, Lupinen, Weidepflanzen wie Luzerne, Gras und Klee, Rasengrün, Straßenbegleitgrün, Heu und Blätter von Bäumen. Ferner können als Ko-Substrate Gülle und Schlachtabfälle verwendet werden.

Vor dem Hintergrund der in Deutschland erfolgenden Müllsortierung und Müllbehandlung beschreibt die DE10021383A1 ein Verfahren und eine Anlage zur Aufbereitung von nicht sortenreinen Reststoffgemengen, die im Wesentlichen aus Biomüll, Holz, Garten- und Lebensmittelabfällen bestehen, und zur Konversion dieser kohlenstoffhaltigen Reststoffe in (marktfähige) Energieträger (Wasserstoff, Biogas, Methanol), elektrischen Strom und Düngemittel. Die zentralen Verfahrensschritte sind die Hochtemperatur-Verschwelung und die Dampfreformierung des Verschwelungsprodukts, nicht aber eine anaerobe bakterielle Vergärung. D.h., bei der beschriebenen Anlage handelt es sich im Wesentlichen um eine Anlage zur chemisch-physikalischen Herstellung von Methanol. Die anaerobe Vergärung bezieht sich lediglich auf einen Nebenprozess des Verfahrens, nämlich auf die Verwertung des aus dem Müll abgeschiedenen Abwassers. Hauptprodukt des beschriebenen Verfahrens ist Methanol und nicht Biogas. Die Biogasanlage stellt ein untergeordnetes Subsystem des Anlagenkonzepts dar, das nicht Stroh vergärt, sondern lediglich aus dem Müll abgeschiedenes Abwasser.

Der vorliegenden Erfindung liegen die Ideen für eine Biogasanlage und für ein Verfahren zur Erzeugung von Biogas zugrunde, die das Problem lösen, geeignete Gärsubstrate in ausreichenden Mengen insbesondere für größere, im industriellen Maßstab arbeitende Anlagen zu wirtschaftlichen Bedingungen zu beschaffen und zu verarbeiten.

Diese Ideen werden mit einer Biogasanlage mit den Merkmalen des Anspruchs 1 und mit dem Verfahren mit den Merkmalen des Anspruchs5 realisiert. Vorteilhafte Weiterbildungen sind in den abhängigen Ansprüchen angeben.

Erfindungsgemäß ist bei der Biogasanlage eine Einrichtung zum mechanischen Aufschluss und ggf. chemischen oder wie hierin beschrieben thermischen Aufschluss von Stroh vorgesehen. Durch den Aufschluss von Stroh läßt sich entgegen der üblichen Meinung auch mit diesen ein erheblicher Gasertrag erzielen. Dadurch wird die Biogaserzeugung, insbesondere die Biogaserzeugung nach dem Feststoffvergärungsverfahren, für eine neue Klasse nachwachsender Rohstoffe zugänglich, denn Stroh steht in großen Mengen zur Verfügung und ist preisgünstig erhältlich. Das Stroh kann als Ergänzung zur herkömmlichen Frischmasse, wie beispielsweise Silage aus Grünschnitt, Silage aus ganzen Getreidepflanzen etc. verwendet werden und stellt so die Versorgung selbst einer Vielzahl großer und sehr großer Biomasse-Kraftwerke, mit beispielsweise 20 oder mehr Fermentern und einer jeweiligen elektrischen Leistung von über 5.000 kW zu wirtschaftlichen Bedingungen sicher.

Wie hierin beschrieben ist zusätzlich zur Vorrichtung zum mechanischen Aufschluss eine Einrichtung zum thermischen Aufschluss von Stroh vorgesehen, wobei diese vorzugsweise eine Einrichtung zur Sattdampfbehandlung ist. Die Einrichtung zur Sattdampfbehandlung umfaßt vorzugsweise einen Druckbehälter und Mittel, die geeignet sind, im Druckbehälter einen Wasserdampf zu erzeugen, mit einem Druck, der zwischen 20 und 30 bar liegt und einer Temperatur, die zwischen 180°C und 250°C liegt. Eine Sattdampfbehandlung findet bei den beschriebenen Drücken und Temperaturen statt und dauert typischerweise 5 bis 15 Minuten. Die Funktion der Sattdampfbehandlung soll am Beispiel von Weizenstroh beschrieben werden.

Weizenstroh besteht zu etwa 40 % aus Cellulose, zu 23 % aus Arabinoxylan (Hemicellulose) und zu 21 % aus Lignin, wobei alle drei Hauptkomponenten eine dichtgepackte Struktur einnehmen. Das wesentliche Hindernis für die biochemische Verwertung der Cellulose und der Hemicellulose ist das für Mikroorganismen unverdauliche Lignin, das den Bakterien den Weg zur Cellulose und zur Hemicellulose versperrt. Bei der Sattdampfbehandlung werden die Ligninstrukturen aufgeweicht bzw. geschmolzen, aber während der verhältnismäßig kurzen Behandlungsdauer im Wesentlichen nicht aus den Halmen ausgelöst. Nach der Sattdampfbehandlung erstarrt das Lignin wieder. Beim Erstarren des Lignins bilden sich jedoch aufgelockerte tröpfchenartige Ligninstrukturen, die ausreichend Zwischenräume lassen, durch die zunächst wässrigen organischen Säuren und dann die Bakterien an die Cellulose und das Arabinoxylan gelangen können, die diese durch die bekannte anaerobe vierstufige Vergärung zersetzen.

Bei der hier beschriebenen Sattdampfbehandlung wird das Lignin also vornehmlich in seiner mikroskopischen Struktur verändert, es wird aber nicht aus den Strohhalmen herausgelöst. Insbesondere bleibt die Struktur der Strohhalme als solche dabei erhalten. Dies stellt einen Unterschied zur Thennodruckhydrolyse dar, die unter grundsätzlich ähnlichen Bedingungen, jedoch für längere Zeiträume durchgeführt wird, und bei der eine echte Hydrolyse stattfindet, also eine Auflösung vormals fester bzw. trockener Stoffe in Wasser. Durch eine Thermodruckhydrolyse wird die Struktur der Strohhalme aufgelöst, und es ergibt sich eine sirupartige Suspension.

In einer vorteilhaften Ausführungsform ist auch eine Einrichtung zum Häckseln vorhanden. Dies trägt weiter zum Auflösen der Ligninstrukturen bei.

Erfindungsgemäß findet eine Vermahlung des Strohs, vorzugsweise in einer Hammermühle, statt. Diese Aufschlussform zerstört die Ligninstrukturen mechanisch. Eine Vermahlung allein ist jedoch bei der Nutzung des Feststoffvergärungsverfahrens nicht sinnvoll, weil sich sonst ein teigartiger Brei bilden würde, der die Gärmasse verklebt und eine Perkolation verhindert.

Wie hierin beschrieben kann die ggf. vorhandene Einrichtung zur Sattdampfbehandlung mehrere Druckbehälter umfassen, die durch Rohrleitungen miteinander verbunden sind. Auf diese Weise kann eine Mehrzahl von Druckbehältern durch eine einzige Dampfquelle, beispielsweise ein einziges Dampfreservoir, versorgt werden, was die Effizienz insbesondere bei größeren Kraftwerken deutlich erhöht.

In einer vorteilhaften Weiterbildung umfaßt die Einrichtung zum Aufschluss von Stroh einen Behälter zum Einweichen desselben in einer Wasser-Laugen-Lösung, einer Wasser-Säure-Lösung, Perkolat oder Gülle. Dieses Einweichen stellt ein Beispiel für den eingangs genannten chemischen Aufschluss dar. Durch dieses Einweichen wird eine (schwach aerobe) Vorhydrolyse initiiert, die vor dem Einbringen in den Fermenter stattfindet. Dazu kann das eingeweichte Stroh nach der Einweichung und vor dem Einbringen in den Fermenter vorzugsweise noch auf 30 bis 50° erwärmt werden. Diese Erwärmung kann mit dem Transport des Strohs von der Einrichtung zum Aufschluss zum Fermenter kombiniert werden, wie unten näher erläutert wird. Durch diese Vorhydrolyse beschleunigt sich die anschließende anaerobe bakterielle Fermentation im Fermenter nochmals.

Man beachte, daß der Aufbau der Biogasanlage und das Verfahren zur Erzeugung von Biogas mit den hier beschriebenen Aufschlussverfahren ohne die Zugabe von zusätzlichen Hefen, Pilzen oder Enzymen auskommt. Tatsächlich wird das ligninhaltige Material allein der Autohydrolyse und der bakteriellen Hydrolyse überlassen. Die Verwendung von Bakterien statt Hefen, Pilzen oder Enzymen ist wesentlich wirtschaftlicher, da die chemischen und biochemischen Prozesse bei der bakteriellen Hydrolyse schneller ablaufen als bei der enzymatischen. Außerdem werden gegenüber dem externen Einsatz von Enzymen und/oder Säuren erhebliche Kosten für deren Beschaffung und Handhabung eingespart.

Eine wichtige Weiterbildung der Erfindung besteht in der Weise, wie der zusätzliche Verfahrensschritt des Strohaufschlusses in den Betrieb der Biogasanlage integriert wird. Die Einrichtung zum Aufschluss des Strohs lohnt sich wirtschaftlich vor allem dann, wenn der Durchsatz der Anlage, bzw. des Biomasse-Kraftwerks hoch ist. Gegenwärtig bekannte Biomasse-Kraftwerke zur Feststoffvergärung sind jedoch im Gegenteil meistens sehr klein und auf den ländlichen Raum beschränkt. Sie verfügen über zwei bis sechs kleinere Fermenter und erreichen eine effektive elektrische Leistung von lediglich 100 bis 700 kW. Dies liegt zum einen daran, daß die Trockenfermentation gemeinhin als noch nicht marktreif angesehen wird, zum andern aber auch an der gestuften Mindestvergütung des EEG für eingespeisten Strom aus NawaRo-Anlagen, die bei der Überschreitung einer Grenze von 500 kW um bis zu 15 % abfallt. Außerdem spricht derzeit gegen eine größere Auslegung von Biogasanlagen mit Trockenfermentation, daß der erforderliche Nachschub an Gärsubstrat gemäß den Anforderungen der Financiers über Jahre im Voraus gesichert sein muß, und daß sich die Betreiber, bei denen es sich typischerweise um Landwirte handelt, auf die von ihnen selbst erzeugbaren NawaRos verlassen wollen.

Durch die erfindungsgemäße Biogasanlage und das erfindungsgemäße Verfahren, die die Verwendung von Stroh gestatten, kann die Versorgung mit Gärsubstrat jedoch auch für weit größere Anlagen sichergestellt werden, da beispielsweise Stroh beim Getreideanbau in weit größeren Mengen anfällt, als es derzeit benötigt wird, und da es sich mit entsprechender (Groß-)Technik auch über größere Strecken verhältnismäßig wirtschaftlich transportieren läßt. Andererseits lohnen sich die Investitions- und Betriebskosten für eine Einrichtung zum Aufschließen des Strohs umso mehr, je größer der Durchsatz der Biogasanlage ist. Ein inhaltlicher Zusammenhang zwischen der Möglichkeit zum Aufschluss des Strohs und der Größe der Biogasanlage besteht insofern, als die Einrichtung zum Aufschluss des Strohs wesentlich dazu beiträgt, die Versorgung auch größerer Biogasanlagen mit Gärsubstrat sicherzustellen, und andererseits die Größe der Biogasanlage und die Verwendung der preisgünstigen Substratart Stroh die Investition für die Einrichtung zum Aufschluss im Besonderen und das große Biomasse-Kraftwerk als Ganzes erst wirtschaftlich macht.

Bei einer bisher nicht bekannten Größe von Biogasanlagen mit beispielsweise 15 bis 30 gro-ßen Garagenfermentern muß der Betrieb der Biogasanlage, und insbesondere der Transport des Gärsubstrats und der Gärreste effizient gestaltet werden. Eine weitere Aufgabe besteht darin, den oben beschriebenen Aufschluss des Strohs in den Betriebsablauf der Biogasanlage zu integrieren.

Wie hierin beschrieben sind die Einrichtungen zum Aufschluss des Strohs in einem Anliefer- und Verladebereich der Biogasanlage untergebracht. Der Anliefer- und Verladebereich umfaßt vorzugsweise stationäre Fördertechnik, die geeignet ist, Frischmasse aus dem Anliefer- und Verladebereich zu einem Fermentervorplatz zu fördern, von dem aus eine Mehrzahl von Fermentern des Garagentyps zugänglich ist. Während bei herkömmlichen Biogasanlagen die Frischmasse und die Gärreste mit einem Radlader transportiert werden, ist nach dieser Weiterbildung stationäre Fördertechnik vorgesehen, durch die sich auch große Mengen an Frischmasse effizient zum Fermentervorplatz transportieren lassen, um von dort in die Fermenter eingebracht zu werden. Auch gestattet eine derartige stationäre Fördertechnik es, die gesamte Biogasanlage einzuhausen, wodurch eine Geruchsbelästigung der Umwelt vermieden wird und es möglich wird, die Biogasanlage auch in der Nähe von Wohngebieten zu betreiben.

Wenn die Biogasanlage komplett eingehaust ist, stellt der Anliefer- und Verladebereich gewissermaßen eine Schnittstelle zwischen dem eingehausten Innenbereich der Anlage und dem Außenbereich dar, und ist somit in einem äußeren Abschnitt der Anlage angeordnet. Der Fermentervorplatz hingegen ist aus logistischen Gründen zentral in der Anlage angeordnet. Durch die stationäre Fördertechnik kann die Frischmasse bzw. das Gärsubstrat vom Anlieferund Verladebereich zum Fermentervorplatz gebracht werden, ohne daß Transportfahrzeuge dazu benötigt würden, die Abgas innerhalb des Einhausungsbereichs erzeugen würden und die außerdem die Betriebskosten erhöhen würden. Vorzugsweise herrscht im Anliefer- und Verladebereich ein leichter Unterdruck, so daß auch beim Anliefern von Frischmasse und beim Verladen von Gärresten nur wenig Luft nach außen dringt, und somit die Geruchsbelästigung minimal ist.

Wie hierin beschrieben befindet sich in dem Anliefer- und Verladebereich mindestens ein eingehauster Anlieferbunker für Frischmasse. Ferner sind vorzugsweise erste Fördermittel vorgesehen, die geeignet sind, Frischmasse aus dem mindestens einen Anlieferbunker für Frischmasse zu einem Frischmassebunker zu befördern. Diese ersten Fördermittel können beispielsweise Förderschnecken, Elevatoren und Förderbänder umfassen, auf denen die Frischmasse aus verschiedenen Anlieferbunkern zum Frischmassebunker befördert werden. Dies hat den Vorteil, daß die Frischmasse bereits dadurch, daß sie aus unterschiedlichen Anlieferbunkern auf ein und denselben Haufen gegeben wird, durchmischt wird, so daß ein späteres Durchmischen der Frischmasse sich erübrigt, bzw. nicht mehr so intensiv vorgenommen werden muß. Bei dieser beschriebenen Frischmasse handelt es sich nicht um das aufzuschließende Stroh , sondern um zusätzliche Frischmasse, wie sie in bisher bekannten Biogasanlagen mit Feststoffvergärung verwendet werden.

Ferner umfaßt der Anliefer- und Verladebereich zweite Fördermittel, insbesondere ein Schubschild, welche geeignet sind, die Frischmasse durch den Frischmassebunker hindurch in Richtung auf den Fermentervorplatz zu befördern. Der Frischmassebunker hat dabei eine zweifache Funktion: zum einen dient er als Transportweg vom Anlieferbereich zum Fermentervorplatz, zum anderen dient er als Zwischenspeicher für Frischmasse. Wichtig ist dabei, daß die Frischmasse, die als erstes in den Frischmassebunker eingebracht wurde, diesen auch als erstes verläßt. Das bedeutet, daß die Frischmasse, die auf den Fermentervorplatz geliefert wird, stets etwa gleich alt und damit gleich stark vorhydrolisiert ist. Damit ergibt sich eine Substrat-Konstanz, die für die anschließende Vergärung vorteilhaft ist.

Außerdem umfaßt der Anliefer- und Verladebereich eine Entladungsstelle für Stroh, und insbesondere für Stroh in Ballenform. In der Entladungsstelle ist ein Kran vorgesehen, der geeignet ist, Ballenmaterial zu greifen und zu befördern.

In dem Anliefer- und Verladebereich sind übrigens wie oben erwähnt die Einrichtungen zum Aufschluss von Stroh vorgesehen, die von einer der eingangs beschriebenen Arten sind .

Wie beschrieben sind dabei dritte Fördermittel, insbesondere Rollenförderer oder Schubförderer vorgesehen, die geeignet sind, einzelne Ballen oder Pakete von Ballen entlang eines Ballenkanals zum Fermentervorplatz zu befördern.

Erfindungsgemäß wird das Stroh durch Vermahlen mechanisch aufgeschlossen, wodurch die Ligninstrukturen ebenfalls aufgerissen werden. Wenn beispielsweise vermahlenes Stroh in eine Naßfermentationsanlage eingeführt würde, würde sich nach Untersuchungen des Erfinders entgegen der in der Fachwelt verbreiteten Ansicht daraus ein erheblicher zusätzlicher Gasertrag ergeben. Durch die Vermahlung werden die flächigen Ligninstrukturen nämlich ebenfalls zerstört. Die Cellulose und das Arabinoxylan können dann durch die in der Gärmasse enthaltenen wässrigen organischen Säuren aufgelöst werden, welche auch in der in Naßfermentationsanlagen typischerweise vorhandenen Gülle und in noch stärkerem Maße in reinen NawaRo-Nassanlagen, die ohne Gülle arbeiten, enthalten sind. Dadurch wird bislang als untauglich erachtete Biomasse für die anaeroben methanerzeugenden Bakterien zugänglich.

Für den ggf. stattfindenden thermischen Aufschluss ist eine Einrichtung zur Thermodruckhydrolyse vorgesehen, mit der das Material einer Thermodruckhydrolyse unterzogen werden kann. Die Thermodruckhydrolyse findet unter ähnlichen Bedingungen statt, wie die eingangs beschriebene Sattdampfbehandlung, jedoch für eine längere Zeitdauer von beispielsweise 60 bis 120 Minuten. Bei der Thermodruckhydrolyse wird das Lignin vollständig ausgelöst, so daß sich eine sirupartige Suspension bildet. Außerdem findet eine echte Hydrolyse, das heißt, eine Aufspaltung von Polymeren in Monomere durch physikalische Einwirkung von Wasser und Hitze, statt. Die sirupartige Suspension kann dann in den Fermentationsprozeß (zurück-)gegeben werden oder an Unternehmen verkauft werden, die aus diesem Material Kraftstoffe der zweiten Generation herstellen. Dabei wird auch der Ligninanteil verwertet sowie weitere, unverwertete organische Substanz. Dies ist bei einer anaeroben, bakteriellen Vergärung nicht möglich.

Die Figuren zeigen:
- Fig. 1: einen Aufriß eines Biomassekraftwerks von Westen betrachtet,
- Fig. 2: einen Aufriß des Biomassekraftwerks von Fig. 1 von Norden betrachten,
- Fig. 3: einen Aufriß des Biomassekraftwerks von Fig. 1 von Süden betrachtet,
- Fig. 4: einen Aufriß des Biomassekraftwerks von Fig. 1 von Osten betrachtet,
- Fig. 5: eine Querschnittsansicht des Biomassekraftwerks von Fig. 1 in westlicher Ansicht,
- Fig. 6: einen Grundriß des Erdgeschosses des Biomassekraftwerks von Fig. 1,
- Fig. 7: einen vergrößerten Ausschnitt aus dem Grundriß von Fig. 6, der eine Strom- und Wärmeerzeugungsanlage zeigt,
- Fig. 8: einen vergrößerten Ausschnitt aus der Grundrißdarstellung von Fig. 6, der einen Anliefer- und Verladebereich zeigt,
- Fig. 9: einen Grundriß des Obergeschosses des Biomassekraftwerks von Fig. 1,
- Fig. 10: eine schematische Darstellung zweier Ansichten eines perforierten Strohballens,
- Fig. 11: eine schematische Querschnittsansicht einer Einrichtung zur Sattdampfbehandlung,
- Fig. 12: eine schematische Querschnittsansicht einer weiteren Einrichtung zur Sattdampfbehandlung, die für loses ligninhaltiges Material bestimmt ist,
- Fig. 13: eine schematische Querschnittsansicht einer Einrichtung zur Sattdampfbehandlung, die zur Sattdampfbehandlung von ballenförmigem Material bestimmt ist,
- Fig. 14: eine schematische Querschnittsansicht einer Einrichtung zur Sattdampfbehandlung, die eine Mehrzahl von Druckbehältern umfaßt.

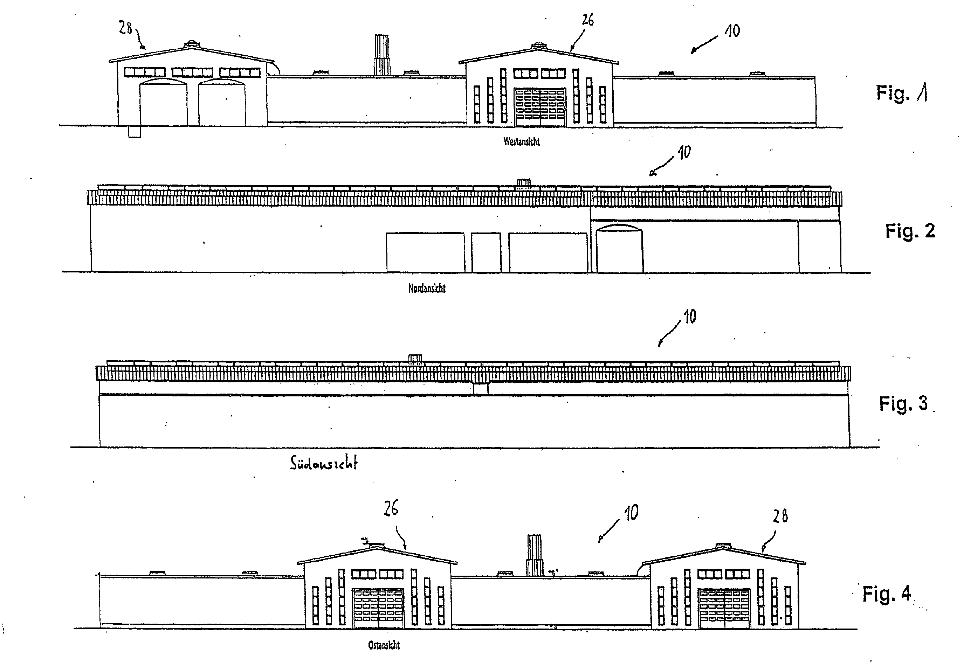

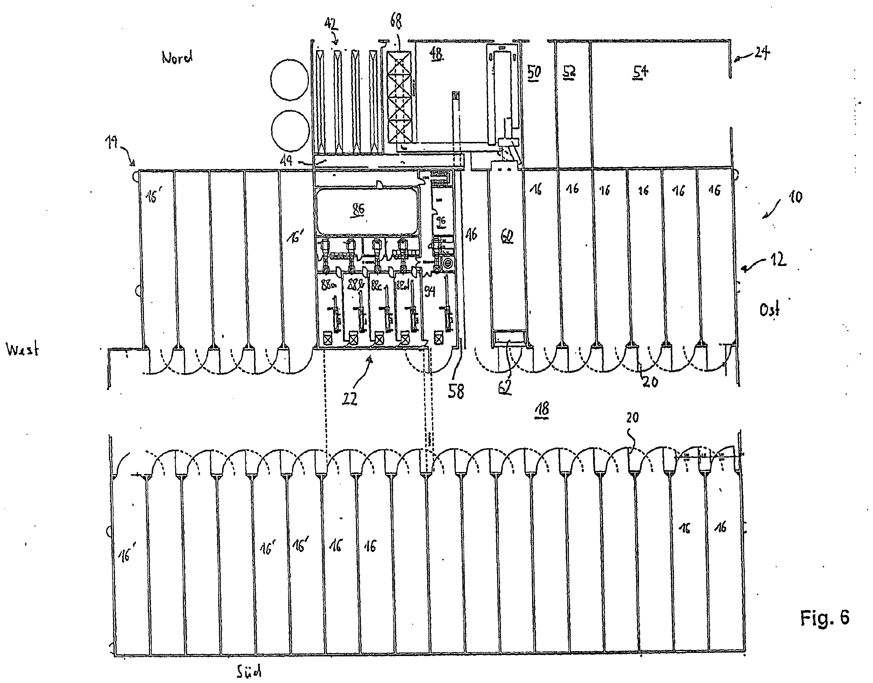

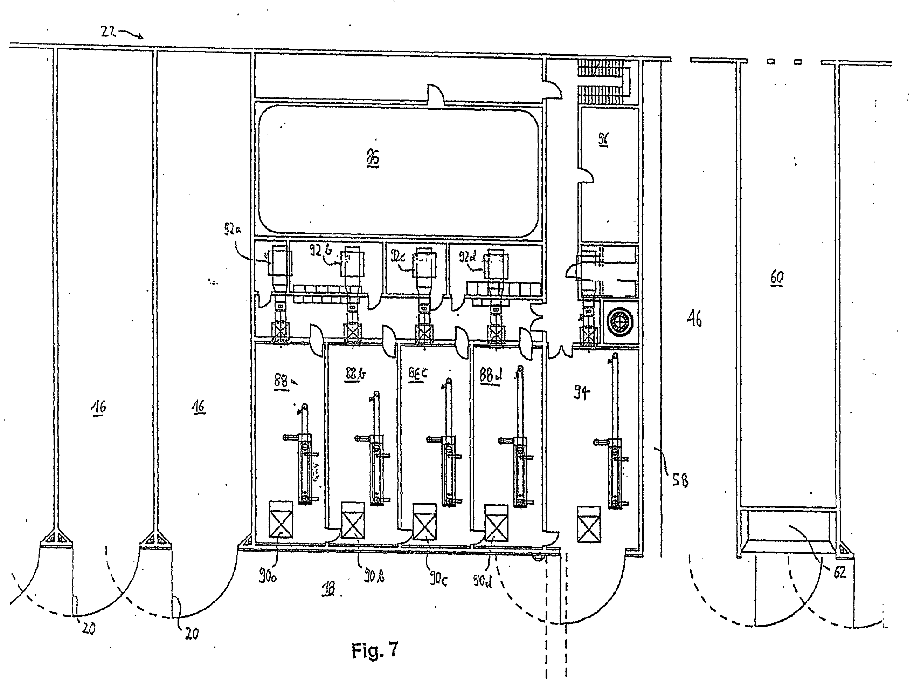

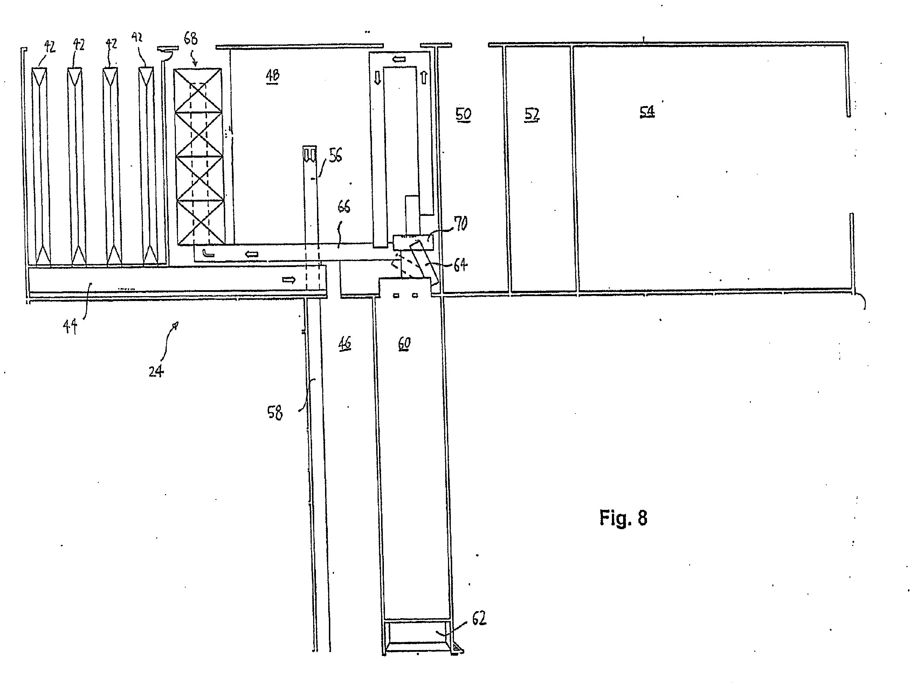

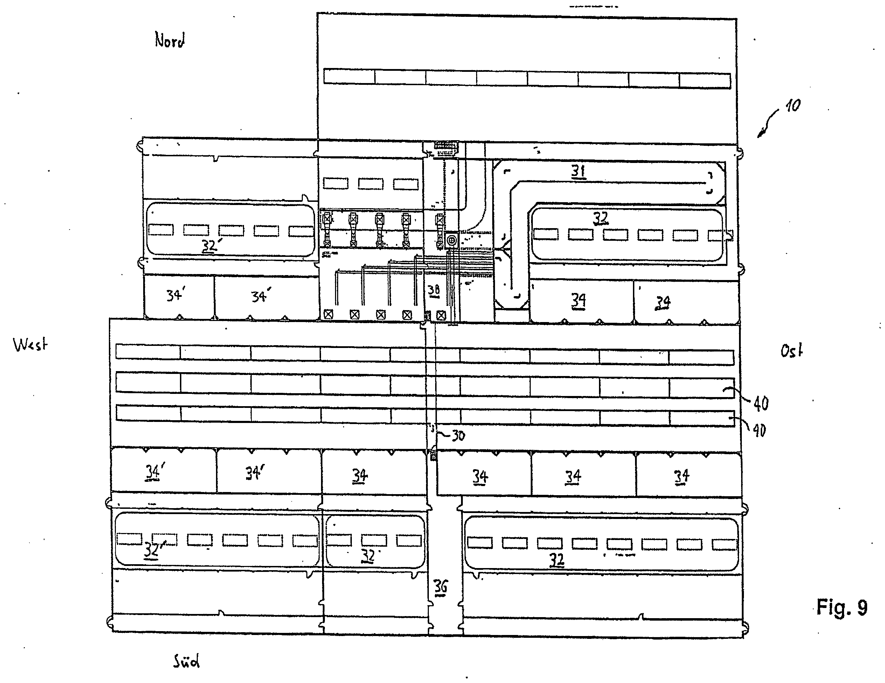

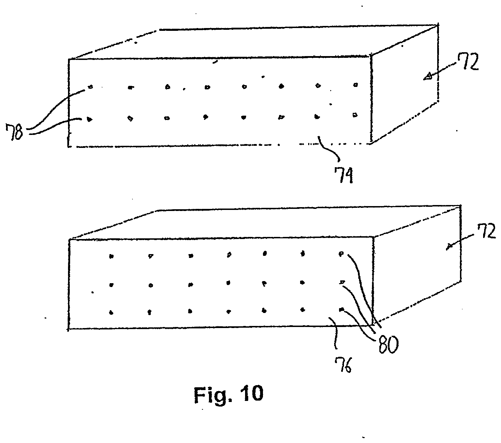

Im Folgenden wird ein Biomassekraftwerk (BMKW) 10 als Beispiel einer Biogasanlage im Detail beschrieben. Dabei zeigen die Fig. 1 bis 4 vier Außenansichten des BMKW 10 und Fig. 5 einen Querschnitt desselben. In Fig. 6 ist der Grundriß des Erdgeschosses des BMKW 10 gezeigt. Fig. 7 zeigt einen vergrößerten Teilbereich des Grundrisses von Fig. 10, in dem eine Strom- und Wärmeerzeugungsanlage des BMKW gezeigt ist. Fig. 8 zeigt einen anderen Teilausschnitt des Grundrisses von Fig. 6, in dem ein Anliefer- und Verladebereich vergrößert dargestellt ist. Fig. 9 zeigt eine Grundrißansicht des Obergeschosses des BMKW 10.

Unter Bezugnahme auf den Grundriß von Fig. 6 gliedert sich das BMKW 10 in einen Grundabschnitt 12 und einen Ausbauabschnitt 14. Der Grundabschnitt 12 umfaßt 18 Fermenter des Garagentyps, die in zwei Reihen angeordnet sind, in der Darstellung von Fig. 5 in einer nördlichen und einer südlichen Reihe. Zwischen den beiden Reihen von Fermentern 16 befindet sich ein Fermentervorplatz 18, zu dem sich die Tore 20 der Fermenter 16 öffnen. Man beachte, daß der Übersicht halber nicht alle Fermenter 16 und Fermentertore 20 in den Figuren mit Bezugszeichen versehen sind.

Ferner umfaßt der Grundabschnitt 12 eine Strom- und Wärmeerzeugungsanlage 22, die in Fig. 7 vergrößert dargestellt und unten näher im Detail beschrieben wird. Außerdem umfaßt der Grundabschnitt 12 einen Anliefer- und Verladebereich 24, der in Fig. 8 vergrößert dargestellt ist und ebenfalls unten näher beschrieben wird.

Wie den Fig. 1 bis 6 zu entnehmen ist, ist der gesamte Grundabschnitt 12 durch eine Hallenstruktur eingehaust, zu der insbesondere ein Fermentervorplatz (FVP)-Hallenabschnitt 26 und ein Anliefer- und Verladebereichshallenabschnitt 28 gehört, wie insbesondere in Fig. 1, 4 und 5 gut zu erkennen ist. Die gesamte Hallenkonstruktion oder Einhausung des Grundabschnitts 12 wird durch eine große zentrale Entlüftungsanlage entlüftet, so daß im Inneren der Hallenkonstruktion stets ein leichter Unterdruck gegenüber dem Atmosphärendruck herrscht.

Der Ausbauabschnitt 14 besteht im Wesentlichen aus 11 zusätzlichen Fermentern 16' und einer Verlängerung des FVP-Hallenabschnitts 26. Der Ausbauabschnitt 14 dient dazu, bei Bedarf bis zu 11 zusätzliche Fermenter 16' vorzusehen. Das bedeutet, daß das BMKW 10 zunächst ohne den Ausbauabschnitt 14 gebaut und in Betrieb genommen würde. Im Betrieb stellt sich dann heraus, ob die vorhandenen 18 Fermenter 16 des Grundabschnitts 12 ausreichend Biogas erzeugen, um die vier Gasmotoren (nicht gezeigt), die für das BMKW 10 bestimmt sind, unter Volllast mit Gas zu versorgen. Falls dies nicht der Fall ist, kann die entsprechende Anzahl Fermenter 16' im Ausbauabschnitt 14 ergänzt werden, der somit auch kleiner ausfallen kann als in Fig. 6 gezeigt. Mit anderen Worten hat das BMKW 10 einen modularen Aufbau, der zum Erreichen einer optimalen Endkonfiguration vorteilhaft ist, weil der exakte Biogasertrag von einer Vielzahl von Faktoren, darunter die Beschaffenheit der vorhandenen Frischmasse, abhängt und nicht theoretisch exakt vorhersagbar ist.

Die nördliche und die südliche Fermenterreihe sind durch eine Technikbrücke 30 verbunden, die insbesondere in Fig. 5, 6 und 9 zu sehen ist. Die Technikbrücke 30 überspannt den FVP 18 in einer Höhe, die gestattet, daß Radlader, von denen in Fig. 5 zwei exemplarisch gezeigt sind, unter ihr selbst mit voll ausgefahrener Ladeschaufel hindurch fahren können, ohne daß sie die Technikbrücke berühren und beschädigen können.

Unter Bezugnahme auf Fig. 9 umfaßt das Obergeschoß des BMKW 10 drei FolienGasspeicher 32 im Grundabschnitt 12 und zwei weitere Foliengasspeicher 32' im Ausbauabschnitt 14. Die Foliengasspeicher 32 sind in den Querschnittsansichten von Fig. 5 und 15 gut zu erkennen. Sie nehmen auf nachfolgend näher beschriebene Weise das Biogas auf, welches in den Fermentern 16 bzw. 16' erzeugt wird.

Ferner umfaßt das Obergeschoß fünf Perkolatumlauftanks (PUT) 34 im Grundabschnitt 12 und vier PUTs 34' im Ausbauabschnitt 14, die ebenfalls in den Querschnittsansichten von Fig. 5 gut zu sehen sind. Ein PUT 34 ist jeweils oberhalb von drei Fermentern 16 angeordnet und empfängt von ihnen Perkolat, welches am Boden der Fermenter gesammelt und in den PUT 34 gepumpt wird. Mit dem Begriff "Perkolat" bezeichnet man den flüssigen, gülleähnlichen Bestandteil der Gärsubstanz.

Ferner befindet sich im Obergeschoß ein Abgas-Abkühlraum 31, ein südlicher Technikraum 36 und ein nördlicher Technikraum 38, die durch die Technikbrücke 30 miteinander verbunden sind. Im FVP-Hallenabschnitt 26 und im Anliefer- und Verladebereichshallenabschnitt 28 sind ferner Lichtbänder 40 angeordnet.

Nachdem ein grober Überblick über die Bestandteile des BMKW 10 gegeben wurde, werden im Folgenden die einzelnen Abschnitte und Komponenten sowie deren Betriebsweise im Detail beschrieben.

### 1. Fermentervorplatz

Der Fermentervorplatz (FVP) 18 ist im Zentrum den BMKW 10 angeordnet. Er dient als Transportweg für Frischmasse zu den jeweiligen Fermentern 16, 16' bzw. von Gärrestesubstanz aus den Fermentern 16, 16'. Außerdem dient er als Anmischfläche, auf der der Inhalt eines Fermenters ausgebreitet wird, von dem etwa ein Fünftel bis ein Viertel als Gärrest entnommen wird und zum Ausgleich dieser Entnahme und des durch die Vergasung entstehenden Masseverlusts etwa ein Drittel durch Frischmasse ergänzt und mit der alten Gärmasse vermischt wird. Diese Arbeit kann auf dem FVP 18 von einem großen Radlader verrichtet werden, wie er in Fig. 5 schematisch dargestellt ist. In der Mitte des FVPs 18 befindet sich eine mit einem Gitterrost versehene Ablaufrinne, in die Sickersäfte und freigesetztes Perkolat fließen. Auf der Höhe der Technikbrücke 30 hat die Ablaufrinne einen Sammelschacht (nicht gezeigt) aus dem die anfallenden Flüssigkeiten über eine Perkolat-Ringleitung (nicht gezeigt) zu einem der PUTs 34 gefördert werden.

### 2. Anliefer- und Verladungsbereich

Der Anliefer- und Verladungsbereich 24 ist in Fig. 8 im Grundriß vergrößert dargestellt. Im gezeigten Ausführungsbeispiel wird, was die Anlieferung betrifft, zwischen loser Frischmasse und Strukturfrischmasse bzw. Ballenfrischmasse unterschieden. Für die lose Frischmasse sind in der gezeigten Ausführungsform vier Anlieferbunker 42 vorgesehen, die durch den Anliefer- und Verladebereichshallenabschnitt 28 eingehaust sind. Ein LKW kann rückwärts in den eingehausten Anlieferbunker rangieren und die Frischmasseladung dort in die Anlieferbunker 42 abkippen oder abschieben. Da im gesamten Anliefer- und Verladebereich 24 ein leichter Unterdruck herrscht, treten kaum störende Gerüche aus der Einhausung nach außen aus. Jeder Anlieferbunker 42 hat einen nach unten konisch zulaufenden Boden, an dessen tiefster Stelle eine oder mehrere Doppelförderschnecke (nicht gezeigt) vorgesehen sind, die die Frischmasse horizontal bis zu einem Becher-Elevator (nicht gezeigt) fördert, welcher die Frischmasse auf ein Förderband 44 befördert, oder direkt auf ein tiefergelegenes Förderband.

Von dem Förderband 44 wird die Frischmasse in einen Frischmassebunker 46 fallengelassen. Da die Frischmasse aus vier oder mehr verschiedenen Bunkern auf demselben Förderband 44 befördert wird und auf denselben Haufen in dem Frischmassebunker 46 geworfen wird, wird die Frischmasse automatisch durchmischt.

Der Frischmassebunker 46 ist eine längliche Kammer, die den Anliefer- und Verladebereich 24 mit dem Fermentervorplatz 18 verbindet, wie insbesondere in Fig. 6 zu sehen ist. Der Frischmassebunker 46 hat eine Fußbodenheizung, mit der die Frischmasse bereits auf eine Temperatur von 42°C erwärmt wird, damit die Gärmasse innerhalb eines Fermenters 16, 16', welche durch die Frischmasse ergänzt wird, durch diese nicht abgekühlt wird, so daß der Gärprozeß nach Verschließen des Fermenters 16 wieder schnell startet und ggf. auch bereits eine schwach aerobe Vorhydrolyse stattfinden kann, die die Gärzeit verkürzt und die Anlagenleistung (Durchsatz an Gärsubstrat) sowie die Wirtschaftlichkeit der Anlage erhöht.

Der Frischmassebunker 46 hat eine doppelte Funktion. Zum einen dient er als Zwischen- oder Pufferspeicher für lose Frischmasse. Zum anderen dient er als Transportweg zwischen dem Anliefer- und Verladebereich 24, also der Peripherie des BMKWs 10, und dem zentral gelegenen Fermentervorplatz 18. Zum Transport ist in dem Frischmassebunker 46 ein Schubschild bzw. Schieber angeordnet (nicht gezeigt) der jeweils neu von oben eingeschüttete lose Frischmasse in Richtung auf den Fermentervorplatz 18 schiebt. Dann wird der Schieber zurückgefahren, um Platz für neue Frischmasse zu machen. Durch diesen Schiebemechanismus wird erreicht, daß die Frischmasse ungefähr in der gleichen Reihenfolge, in der sie am Eingang in den Frischmassebunker 46 eingeworfen wurde, auch auf der Seite des Fermentervorplatzes 18 aus ihm herausgeschoben wird. Dies bedeutet, daß die Frischmasse, die zum Fermentervorplatz 18 gelangt, immer ungefähr gleich alt und daher von konstanter Beschaffenheit ist, was für die anschließende Vergärung vorteilhaft ist.

Ferner umfaßt der Anliefer- und Verladebereich 24 einen Abschnitt für das Anliefern und Transportieren von Struktur- bzw. Ballenmaterial, insbesondere für Stroh. Dieser Abschnitt zum Anliefern und Transportieren von Ballenmaterial umfaßt einen Bereitstellungsraum 48, einen Ballen-Anlieferungsraum 50, einen Aufschlussbereich 52 und ein Zwischenlager 54. Im Folgenden wird dieser Bereich des Anliefer- und Verladebereichs 24 unter Bezugnahme auf Stroh als stark lignifiziertes, ballenförmiges Strukturmaterial beschrieben, aber es versteht sich, daß dieser Abschnitt auch zur Anlieferung, Bearbeitung und den Weitertransport von anderem ballenförmigen Strukturmaterial verwendet werden kann.

Ein Kran (nicht gezeigt) ist an einer Laufschiene so angebracht, daß er Strohballen in jedem der Räume 48 bis 54 aufgreifen und ablegen kann. Die Strohballen werden im Strohanlieferungsraum 50 angeliefert und von dem Kran (nicht gezeigt) in das Zwischenlager 54 transportiert. Bevor das Stroh zum Fermentervorplatz 18 befördert wird, wird es in dem Aufschlussbereich 52 vorbehandelt, nämlich aufgeschlossen. Der Aufschluss des Strohs ist nötig, da das Stroh stark lignifiziert ist, und die Bakterien im Fermenter 16 aufgrund der ligninkrustierten Cellulose nur sehr schlecht an die vom Lignin eingeschlossenen Nährstoffe gelangen. In dem Aufschlussbereich 52 kann das Stroh je nach Ausführungsform des BMKW 10 auf unterschiedliche Arten aufgeschlossen werden. Beispielsweise kann das Stroh chemisch aufgeschlossen werden, indem es in einem Behälter eingeweicht wird, welcher Wasser, eine Wasser-Laugen-Lösung oder eine Wasser-Säure-Lösung enthält. Durch die Einweichung wird das Lignin, das die Cellulose weitgehend eingeschlossen hat, teilweise aufgelöst. Nach der Entnahme aus dem Behälter ist die Cellulose nicht mehr hinter einer Ligninkruste verborgen, sondern für die Hydrolyse und die Bakterien zugänglich. Dadurch wird das Stroh, welches in herkömmlichen Naß- oder Trockenvergärungsanlagen bisher nur als Strukturmaterial verwendet wird, zu einem wertvollen Gärsubstrat, welches zur Biogasentwicklung wesentlich beiträgt.

Erfindungsgemäß wird das Stroh in dem Aufschlussbereich 52 mechanisch unter Verwendung einer Hammermühle aufgeschlossen oder wie hierin beschrieben indem es einem Thermodruck ausgesetzt wird, d.h. bei hohem Druck von beispielsweise 20 bis 30 bar für fünf bis zehn Minuten auf 180°C bis 250°C erhitzt wird. Dabei weicht das Lignin auf. Nach dem Erkalten des Strohs erstarrt das Lignin zwar wieder, jedoch in Form sehr kleiner Kügelchen mit Zwischenräumen dazwischen, die den Weg für die autohydrolytischen, organischen Säuren und für die anaeroben Bakterien zu den Nährstoffen des Strohs freigeben. Ein weiteres Beispiel ist eine Erweiterung der Thermodruckbehandlung, bei der der Druck in dem entsprechenden Behälter plötzlich reduziert wird, wodurch das Wasser in den Strohstrukturen verdampft und sich sehr rasch ausdehnt. Dabei werden die Ligninstrukturen zerrissen und die Nährstoffe für die anaeroben Bakterien freigelegt. Die weiteren Details des Strohaufschlusses werden im nachfolgenden Abschnitt angegeben.

Im Bereitstellungsraum 48 ist ein Rollenförderer 56 vorgesehen, auf den einzelne Strohballen und/oder Strohballenpakete durch den Kran (nicht gezeigt) aufgelegt werden, und der die Strohballen durch einen Strohkanal 58, der parallel zum Frischmassebunker 46 angeordnet ist, zum Fermentervorplatz 18 befördert (siehe Fig. 6).

Wie der obigen Beschreibung zu entnehmen ist, wird sowohl die lose Frischmasse als auch die ballenförmige Frischmasse durch stationäre Fördertechnik von dem Anliefer- und Verladebereich 24 zum Fermentervorplatz 18 befördert. Insbesondere stellen dabei der Frischmassebunker 46 und der Strohkanal 58 die Verbindung zwischen dem zentralen Fermentervorplatz 18 und dem peripheren Anliefer- und Verladebereich 24 bereit, und dieser Transport geschieht vollständig im eingehausten BMKW 10. Der Transport mit der stationären Fördertechnik ist für hohe Durchsätze geeignet und insbesondere schneller, platzsparender und kostengünstiger als eine Anlieferung mit Radladern. Wie Fig. 6 zu entnehmen ist, enden der Strohkanal 58 und der Frischmassebunker 46 an einer zentralen Stelle des Fermentervorplatzes 18, so daß die Wege zwischen dem fermentervorplatzseitigen Ende des Frischmassebunkers 46 bzw. Strohkanals 58 und dem zu beliefernden Fermenter 16 im allgemeinen kurz sind.

Wie oben erwähnt wurde, ermöglicht der Aufschluss des Strohs im Aufschlussbereich 52 es, Stroh trotz seines hohen Ligningehaltes als Gärsubstrat zu verwenden. Dies ist äußerst vorteilhaft, weil Stroh bei der Getreideproduktion ohnehin anfällt, und gar nicht ausreichend Verwendung für dieses besteht. Da das BMKW 10 für nachwachsende Rohstoffe konzipiert ist, bietet es sich an, in der Umgebung des BMKW 10 speziell zur Verwendung im BMKW 10 geeignete Rohstoffe anzubauen, die jedoch im allgemeinen nicht für die Nahrung bestimmt sind. Dies stellt jedoch einen gewissen Ziel-Konflikt dar, weil stets ein bestimmter Anteil von den begrenzten zur Verfügung stehenden Flächen für die Nahrungsproduktion reserviert ist. Die Verwendung von Stroh als Gärsubstrat stellt eine sehr attraktive Lösung dar, da Stroh, welches bei der Getreideproduktion anfällt, die gleichzeitige Produktion von Nahrungsmitteln und kraftwerkstauglicher Biomasse erlaubt.

Stroh hat aber noch einen weiteren Vorteil. Im Allgemeinen ist die Füllhöhe in Fermentern durch den Druck begrenzt, der sich am Fermenterboden ergibt: Dieser Druck muß stets so niedrig sein, daß das Gärsubstrat noch durchlässig für Perkolat ist. Wenn man hingegen, gemäß einer Weiterbildung der Erfindung, in der untersten Lage eines jeden Fermenters 16 eine Schicht aus Strohballen einbringt, kann auf diese Schicht noch die gesamte übliche Füllhöhe an Gärsubstanz geschichtet werden, da die Strohballenschicht auch bei dem dann auftretenden Druck noch durchlässig für Perkolat ist. Diese unterste Strohschicht stellt also eine zusätzliche Menge an Gärsubstrat dar, die in einem Fermenter verwendet werden kann, so daß die Anlagenleistung (Raumleistung gemessen in neuem Substrat pro Fermenter und Tag) erheblich gesteigert wird.

Wie beschrieben werden die Strohballen in Paketen aus acht Strohballen auf den Rollenförderer 56 aufgelegt, die zwei Ballen breit und vier Ballen hoch sind. Diese Pakete werden als Ganzes durch den Strohkanal 58 transportiert und an dessen Ende am Fermentervorplatz 18 von einem Umsetzer (nicht gezeigt) abgehoben und an einen Radlader oder Gabelstapler übergeben, der die Pakete ebenfalls als Ganzes oder in 2 Teilen empfängt und zum Fermenter bringt. Aus diesen Paketen kann relativ einfach und zügig die besagte unterste Strohballenschicht aufgebaut werden.

Wie weiter in Fig. 8 zu sehen ist, ist ein Gärrestebunker 60 vorgesehen, der sich parallel zum Frischmassebunker zwischen dem Fermentervorplatz 18 und dem Anliefer- und Verladebereich 24 erstreckt. Der Gärrestebunker 60 hat an seinem dem Fermentervorplatz zugewandten Ende ein Schütttrog 62 für Gärreste, die den Eingang in den Gärrestebunker 60 bildet. In diesen Schütttrog 62 werden Gärreste von einem Radlader gekippt. Von dort werden sie mittels einer Förderschnecke in den Gärrestebunker gedrückt. Durch das diskontinuierliche Nachdrücken immer neuer Gärreste wird die Masse langsam durch den Gärrestebunker 60 bis zum anderen Ende befördert, wo sie durch weitere Förderschnecken aus dem Gärrestebunker 60 transportiert werden.

Der Gärrestebunker 60 hat eine dreifache Funktion. Zum einen dient er als Transportweg zwischen dem Fermentervorplatz 18 im Zentrum des BMKW 10 und dem Anliefer- und Verladebereich, mit ähnlichen Vorteilen, wie sie im Hinblick auf den Frischmassebunker 46 und den Strohkanal 58 beschrieben wurden. Zum anderen dient der Gärrestebunker 60 aber auch als thermophil betriebener Nachgärer und wirkt quasi wie ein weiterer Fermenter. Daher ist der Gärrestebunker 60 auch an das Biogassystem angeschlossen.

Schließlich dient der Gärrestebunker 60 als Zwischenspeicher für Gärreste. Er ist so dimensioniert, daß er mindestens so viele Gärreste faßt, wie an zwei Tagen anfallen können. Dies ermöglicht es, den Abtransport der anfallenden Gärreste an Wochentagen vorzunehmen, so daß der Abtransport nicht durch LKW-Fahrverbote behindert ist.

Am Austrittsende des Gärrestebunkers 60 ist eine Weiche 64 vorgesehen, die es ermöglicht, die Gärreste entweder direkt über ein Förderband 66 zu Verladesilos 68 zu transportieren, oder den Umweg über eine Dehydriereinrichtung 70 zu nehmen. Im gezeigten Ausführungsbeispiel ist die Dehydriereinrichtung 70 eine Schneckenpresse, die geeignet ist, Wasser bzw. Perkolat aus den Gärresten zu pressen und in einen der PUTs 34 einzuführen. Ob der Umweg über die Dehydriereinrichtung 70 genommen wird, hängt von dem aktuellen Bedarf an Perkolat ab.

Die Verladesilos für Gärreste 68 sind Hochsilos, die auf Trapezgestellen angeordnet sind, so daß ein LKW unter die Silos 68 fahren kann und somit auf einfache Weise beladen werden kann.

In einem Beispiel ist eine konventionelle Trocknungsanlage, beispielsweise ein Trommel- oder Bandtrockner (nicht gezeigt) vorgesehen, die geeignet ist, die Gärreste auf unter 25 % vorzugsweise auf 15% Wasseranteil zu trocknen. Die Wärme für die Trocknungsanlage wird dabei vorzugsweise durch die Abwärme von den Generatorsets bereitgestellt. Ferner ist eine Vergasungsanlage (nicht gezeigt) vorgesehen, in der die getrockneten Gärreste einer sogenannten Holzvergasung unterzogen werden, bei der aus den getrockneten Gärresten durch Pyrolyse oder Teilverbrennung unter Luftmangel brennbares Holzgas (Schwachgas) gewonnen wird. Dieses Holz- bzw. Schwachgas wird nach der Entfernung des anfallenden Teers aus dem Holzgas nach irgend einem bekannten Verfahren in das Biogassystem eingespeist und kann dann völlig unproblematisch als Treibstoff für die Gas-Ottomotoren eingesetzt werden.

Der Energiegehalt des Holzgases reduziert den Bedarf an Biogas um bis zu 20 %, ggf. auch mehr, so daß für dieselbe Stromleistung bis zu 20 % ggf. auch bis zu 30% weniger Substrat für die Vergärung eingesetzt werden muß. Dadurch steigt die Wirtschaftlichkeit der Anlage als Ganzes erheblich.

### 3. Strohaufschluss

Wie eingangs erwähnt, wird bei dem gezeigten Biomasse-Kraftwerk das Stroh im Anlieferund Verladebereich 24 angeliefert und im Aufschlussbereich 52 und eventuell zusätzlich im Bereitstellungsraum 48 aufgeschlossen. In diesem Beispiel wird Stroh als LHNawaRo in Ballenform angeliefert und auch in Ballenform aufgeschlossen, um schließlich in Ballenform in die Garagenfermenter 16 eingebracht zu werden. Die Ballen haben dabei vorzugsweise eine Dichte von über 200 kg/m³, die sich nur mit Höchstdruckballenpressen erzeugen lassen. Der Vorteil einer solch hohen Dichte der Strohballen besteht darin, daß dann die Kapazität eines LKW sowohl bezüglich des zulässigen Gewichtes der Ladung als auch des möglichen Volumens der Ladung optimal ausgeschöpft wird, so daß das Stroh auch über weitere Strecken zu wirtschaftlichen Bedingungen geliefert werden kann.

In dem Beispiel umfaßt die Vorbehandlung zum Aufschluss des Strohs vier Schritte, die in dem Aufschlussbereich 52 bzw. dem Bereitstellungsraum 48 durchgeführt werden, nämlich
1.) Perforieren bzw. Lochen der Strohballen,
2.) Einweichen der Strohballen in Wasser,
3.) Sattdampfbehandlung der eingeweichten Strohballen, und
4.) Einweichen der Strohballen in Perkolat.

Diese Schritte und die dabei eingesetzten Vorrichtungen werden im Folgenden beschrieben. Parallel kann ein Teil des Strohs in die Aufschlussform "Vermahlung" und/oder in die Aufschlussform "Thermodruckhydrolyse" gegeben werden. Die Kombination der verschiedenen Aufschlussformen ist besonders vorteilhaft, denn jede hat ihre Vor- und Nachteile für den Praxisbetrieb. Durch die Kombination wird der beste Gesamteffekt erzielt. Erfindungsgemäß ist ein Schritt des Vermahlens des Strohs, insbesondere zu einer Pulverkonsistenz, vorgesehen, wobei das vermahlene Stroh der übrigen Frischmasse beigemischt werden kann. Das vermahlene Stroh führt zu einem besonders hohen Gasertrag, jedoch ist der Anteil im Verhältnis zur Frischmasse insofern begrenzt, als das durch Perkolat benetzte pulverisierte Stroh eine klebrige Masse bildet, die wegen der Handhabbarkeit mit ausreichend Frischmasse gemischt werden muß.

Vorzugsweise bestehen zwischen 5 und 25 Gewichtsprozente des Gärsubstrates als Ganzes aus gemahlenem Stroh. Vorzugsweise werden zwischen 5% und 35% des insgesamt verwendeten Strohs vermahlen und dadurch mechanisch aufgeschlossen.

Ferner ist es vorteilhaft, unabhängig von der Vorbehandlung des übrigen Strohs 5-20% des insgesamt verwendeten Strohs in einer Thermodruckhydrolyse aufzuschließen und das dabei erhaltene, sirupähnliche Material, das sogenannte "slurry" in den Perkolatkreislauf einzubringen.

Schließlich ist es vorteilhaft, unabhängig von den übrigen Verfahrensschritten zum Strohaufschluss das aus dem Fermenter entnommene Gärsubstrat mechanisch durchzupressen.

### 3.1. Perforation

Die Perforation der Strohballen dient dazu, das Innere des Strohballens für das Einweichen, für die Sattdampfbehandlung und für die nachfolgende Einweichung in Perkolat zugänglich zu machen. In dem Beispiel werden die Strohballen von zwei Seiten gelocht, wie unter Bezugnahme auf Fig. 10 näher erläutert wird.

Fig. 10 zeigt oben eine perspektivische Ansicht eines Strohballens 72, in der auf dessen Unterseite 74 geblickt wird. Die untere Abbildung zeigt eine perspektivische Ansicht desselben Strohballens 72, bei der auf dessen Oberseite 76 geblickt wird. Von der Unterseite 74 aus ist der Strohballen 72 durch einen ersten Satz Löcher 78 perforiert, die nicht durch den gesamten Ballen 72 hindurchgehen. Ferner ist der Strohballen 72 von der Oberseite 76 aus mit einem zweiten Satz Löcher 80 perforiert, die ebenfalls nicht durch den gesamten Strohballen 72 hindurchgehen. Die Löcher 78 und 80 sind so gegeneinander versetzt, daß die Löcher des ersten Satzes 78 und die Löcher des zweiten Satzes 80 durch Materialbrücken voneinander getrennt sind. Durch diese Art der Lochung kann das Einweichwasser des zweiten Schritts, der Sattdampf des dritten Schritts und das Perkolat des vierten Schritts gut in das Innere des Strohballens 72 eindringen, ohne auf der anderen Seite wieder herauszulaufen.

### 3.2. Einweichen

In dem Aufschlussraum 52 von Fig. 8 sind geeignete Behälter zum Einweichen von Strohballen vorgesehen, die in der Darstellung nicht gezeigt sind. Die Größe der Behälter zum Einweichen sind auf die Ausmaße der Strohballen abgestimmt, so daß das Einweichen platzsparend und effizient durchgeführt werden kann.

### 3.3. Sattdampfbehandlung

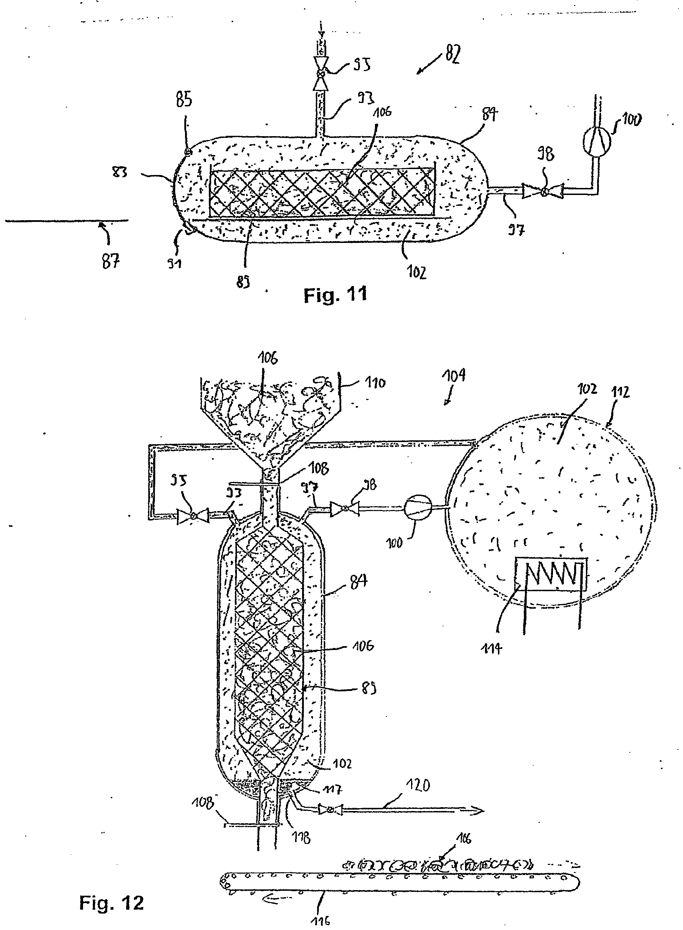

Im Aufschlussbereich 52 oder im Bereitstellungsraum 48 ist eine Einrichtung zur Sattdampfbehandlung vorgesehen. Unter Bezugnahme auf Fig. 11 bis 14 werden verschiedene Einrichtungen zur Sattdampfbehandlung beschrieben, die in der gezeigten Anlage oder einer abgewandelten Anlage verwendet werden können.

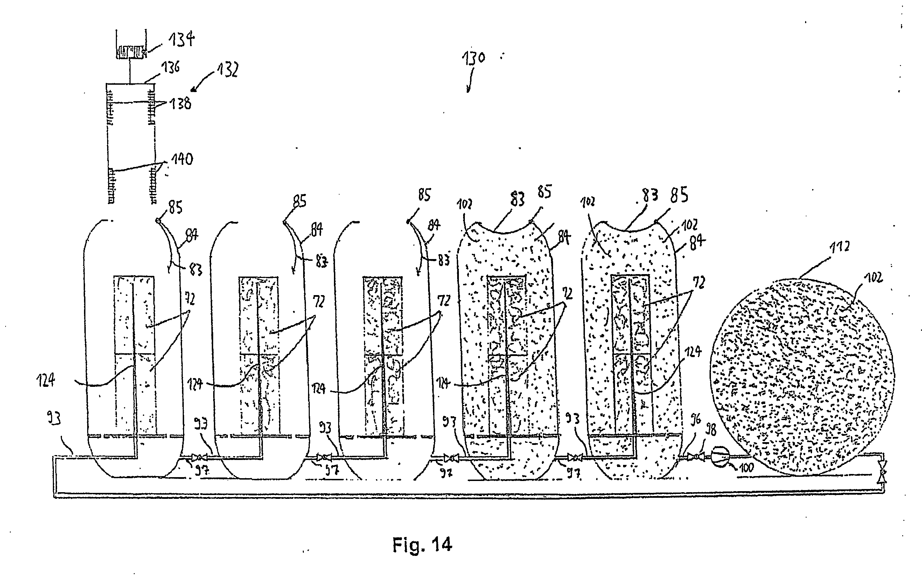

Fig. 11 zeigt in einer schematischen Querschnittsansicht einen einfachen Aufbau einer Einrichtung 82 zur Sattdampfbehandlung. Die Einrichtung 82 umfaßt einen Druckbehälter 84 mit einem Deckel 83, der über ein Gelenk 85 schwenkbar am Druckbehälter 84 angelenkt ist. Eine Zuführeinrichtung für das Stroh ist schematisch angedeutet und durch Bezugszeichen 87 bezeichnet. Falls mit der Einrichtung 82 zur Sattdampfbehandlung Strohballen behandelt werden sollen, kann die Zuführeinrichtung 87 beispielsweise durch ein Förderband oder einen Rollenförderer gebildet werden. Falls die Einrichtung 82 für loses Stroh 106, verwendet werden soll, kann die Zuführeinrichtung 87 aus Schienen bestehen, auf denen ein Behälter 89 für loses Material in den Druckbehälter 84 geschoben werden kann. Der Behälter 89 ist für Wasserdampf durchlässig, aber geeignet, das lose Material zu halten und kann beispielsweise ein oben offener Gitterbehälter bzw. Korb sein. Der Deckel 83 des Druckbehälters 84 kann durch einen Schließmechanismus 91 verschlossen werden. Vorzugweise wird der Deckel 83 zum Öffnen des Druckbehälters 84 nach innen geschwenkt, wie dies beispielsweise in Fig. 14 zu sehen ist, so daß der Deckel 83 durch den Druck im Inneren des Druckbehälters 84 in die geschlossene Stellung gedrückt wird und dadurch einfacher abgedichtet wird.

Der Druckbehälter 84 ist mit einer Zufuhrleitung 93 und einem Zuführungs-Ventil 95 verbunden, durch die Sattdampf 102 mit einem Druck von bis zu 30 bar und einer Temperatur von bis zu 250°C von einem Dampfreservoir (nicht gezeigt) in den Druckbehälter 84 eingeführt werden kann. Ferner ist der Druckbehälter 84 mit einer Auslaßleitung 97 mit einem Auslaß-Ventil 98 verbunden, über die der Dampf nach der Sattdampfbehandlung aus dem Druckbehälter 84 ausgelassen werden kann. Ferner ist in der Auslaßleitung 97 ein Kompressor 100 angeordnet, mit dem der Sattdampf 102 in das Reservoir (nicht gezeigt) zurückbefördert werden kann.

Im Folgenden wird das Verfahren der Sattdampfbehandlung unter Bezugnahme auf die Einrichtung 82 zur Sattdampfbehandlung von Fig. 11 erläutert. Zunächst wird das Stroh 106 als Ballen oder als loses Material in einem Behälter wie in dem Behälter 89 in den Druckbehälter 84 eingebracht und der Druckbehälter 84 geschlossen. Danach wird das Ventil 95 in der Zufuhrleitung 93 geöffnet, so daß heißer Wasserdampf mit einer Temperatur von 180°C bis 250°C und einem hohen Druck von zwischen 20 und 30 bar von einem Dampfreservoir (nicht gezeigt) in den Druckbehälter 84 eingeführt wird. Der eingeführte Sattdampf ist in Fig. 11 schematisch angedeutet und mit dem Bezugszeichen 102 bezeichnet.

Das Stroh 106 wird dem Sattdampf 102 für 5 bis 15 Minuten ausgesetzt. Dabei wird das Lignin in dem Material geschmolzen, aber nicht aus dem Material ausgelöst. Für die Effizienz der Sattdampfbehandlung ist es vorteilhaft, wenn das Stroh in dem oben genannten zweiten Schritt zuvor eingeweicht wurde, weil das Wasser dann bereits im Material vorhanden ist und nur noch erhitzt werden muß, was die Behandlungsdauer verkürzt.

Nach der vorbestimmten Verweildauer von 5 bis 15 Minuten wird der Sattdampf durch die Auslaßleitung 97 aus dem Druckbehälter 84 entlassen. Dieses Entlassen des Drucks findet vorzugsweise schlagartig statt, so daß der Druck innerhalb von 5 Sekunden oder weniger um mindestens 80 % abnimmt. Durch die rasche Absenkung des Drucks verdampft das Wasser in den Strukturen des Strohs schlagartig und dehnt sich dabei rasch aus. Dabei werden die Ligninstrukturen des Strohs zerrissen, so daß die Nährstoffe (Cellulose und Arabinoxylan) für wässrige organische Säuren und die anaeroben Bakterien zugänglich werden.

Nach dem Entlassen des Drucks aus dem Druckbehälter 84 wird das Stroh 106 aus dem Druckbehälter 84 entfernt, und es kühlt sich ab. Beim Abkühlen erhärtet das geschmolzene Lignin wieder. Jedoch bilden sich beim Erstarren des Lignins nicht wieder die ursprünglichen flächigen Strukturen, sondern es koaguliert vielmehr in einer Tröpfchenstruktur, die Zwischenräume läßt, durch die zunächst organische Säuren und dann Bakterien an die Cellulose und das Arabinoxylan (Hemicellulose) gelangen können.

Der in Fig. 11 grundsätzlich gezeigte Aufbau der Einrichtung 82 zur Sattdampfbehandlung kann auf vielfältige Weise modifiziert werden, und einige Beispiele solcher Modifikationen werden im Folgenden angegeben. Dabei werden identische oder funktionell gleiche Komponenten mit denselben Bezugszeichen wie in Fig. 11 bezeichnet, und deren Beschreibung nicht wiederholt.

Fig. 12 zeigt einen Aufbau einer Einrichtung zur Sattdampfbehandlung, die für eine quasi kontinuierliche Verarbeitung von losem Material ausgebildet ist. Auch hier ist im Inneren des Druckbehälters 84 ein Behälter 89 für loses Material 106 vorgesehen, jedoch ist dieser in dem Druckbehälter 84 fest installiert. Zum Befüllen des Behälters 89 wird ein druckfester Schieber 108 geöffnet, so daß das ligninhaltige Material 106 aus einem Trichter 110 in den Behälter 88 fällt. Wenn eine ausreichende Menge Material 106 im Behälter 89 ist, wird der druckfeste Schieber 108 geschlossen, und die Sattdampfbehandlung findet auf dieselbe Weise statt, wie sie unter Bezugnahme auf Fig. 11 beschrieben wurde. Zusätzlich zu den Komponenten von Fig. 11 ist jedoch in Fig. 12 ein Reservoir 112 für Sattdampf 102 gezeigt, welches über eine Heizung 114 verfügt. Nach der Sattdampfbehandlung wird der Dampf über die Auslaßleitung 97 entlassen und über den Kompressor 100 in das Reservoir 112 gedrückt. Danach wird ein weiterer druckfester Schieber 108 am unteren Ende des Behälters geöffnet, und das aufgeschlossene lose Material 106 fällt auf ein Förderband 116 zum Weitertransport.

Am unteren Ende des Behälters 84 sammelt sich insbesondere bei der Thermodruckhydrolyse eine flüssige Masse 117, die man als "Slurry" bezeichnet und die über eine weitere Leitung 118 abgelassen und über eine Leitung 120 in die Perkolatumlauftanks (nicht gezeigt) geleitet wird.

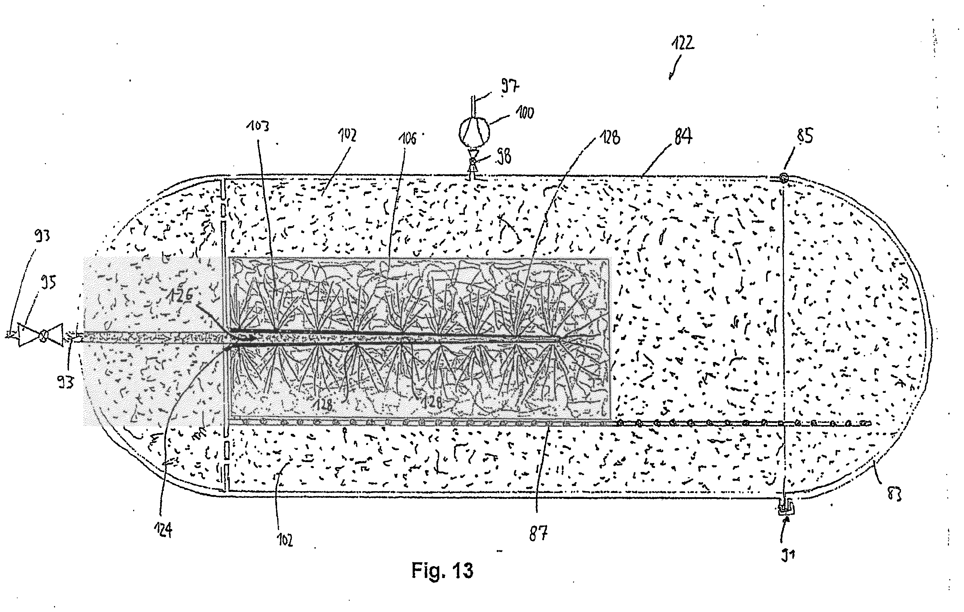

Fig. 13 zeigt eine weitere Ausführungsform 122 einer Einrichtung zur Sattdampfbehandlung, die speziell für die Behandlung von Ballenmaterial, insbesondere Strohballen 72, ausgelegt ist. Der Aufbau ist grundsätzlich ähnlich zum Aufbau von Fig. 11 und wird nicht erneut beschrieben. Der wesentliche Unterschied besteht jedoch darin, daß ein Spieß oder Dorn 124 vorgesehen ist, der einen inneren Hohlraum 126 und mit diesem inneren Hohlraum 126 verbundene düsenartige Öffnungen 128 hat. Der innere Hohlraum 126 ist mit der Zufuhrleitung 93 in Fluidverbindung.

Im Betrieb der Einrichtung zur Sattdampfbehandlung 122 von Fig. 13 wird ein Strohballen 72 bzw. 106 über die Zuführeinrichtung 87, die in der gezeigten Ausführungsform durch eine Rollenbahn gebildet wird, in der Darstellung von Fig. 13 von rechts in den Druckbehälter 84 eingebracht und auf den Spieß 124 aufgespießt. Dann wird der Druckbehälter 84 wie gehabt verschlossen, und der Sattdampf 102 wird über die Zufuhrleitung 93, den inneren Hohlraum 126 des Spießes 124 und die düsenartigen Öffnungen 128 in den Strohballen 72 injiziert. Dadurch wird erreicht, daß auch das Innere des Strohballens 72 wirksam mit dem Sattdampf in Kontakt kommt. Wird nämlich der Sattdampf wie in Fig. 11 gezeigt lediglich von außen an das Material zugeführt, kann es passieren, daß insbesondere bei einem sehr stark gepreßten Ballen 72 der Sattdampf nicht ausreichend mit dem Material im Inneren des Ballens in Kontakt kommt. Statt dessen wird die Luft, die sich im Ballen befindet, durch den unter hohem Druck befindlichen Dampf im Inneren des Ballens komprimiert, möglicherweise ohne sich bei den verhältnismäßig kurzen Behandlungszeiten ausreichend mit dem heißen Dampf zu durchmischen. Durch die Verwendung des Spießes 124 wird eine gründliche Sattdampfbehandlung auch im Inneren des Ballens 72 gewährleistet.

Schließlich zeigt Fig. 14 eine weitere Einrichtung 130 zur Sattdampfbehandlung, die fünf Druckbehälter 84 umfaßt, die ähnlich wie bei der Einrichtung 122 von Fig. 13 mit Spießen 124 versehen sind. Allerdings sind bei der Einrichtung 130 von Fig. 14 die Druckbehälter 84 vertikal angeordnet, so daß die Strohballen von oben mit einem Kran 132 in die Druckbehälter eingeführt werden können 84. Der Kran 132 umfaßt eine Laufkatze 134 und ein Gestell 136, an dem ein oberer Greifer 138 für einen oberen Ballen und ein unterer Greifer 140 für einen unteren Ballen ausgebildet sind. Mit dem Kran 130 können somit zwei vertikal übereinander angeordnete Strohballen 72 gegriffen werden, von oben in den Druckbehälter 84 eingeführt und auf einen Spieß 124 aufgespießt werden, der zu diesem Zwecke etwa doppelt so lang ausgebildet ist wie der Spieß 124 von Fig. 13.

Sämtliche Druckbehälter 84 von Fig. 14 sind über eine Rohrleitung mit demselben Druckreservoir 112 verbunden. Dabei wird ähnlich wie in Fig. 13 der Sattdampf 102 jeweils über die Zuleitung 92 durch den Spieß 124 und durch die Strohballen 72 hindurch in den Druckbehälter 84 eingeführt.

Die Weiterbildung von Fig. 14 ist für eine Anlage mit hohem Durchsatz konzipiert, in der die Sattdampfbehandlung sehr effizient durchgeführt werden kann.

### 3.4 Einweichung in Perkolat oder ähnlichem

In dem vierten oben genannten Verfahrensschritt werden die vorbehandelten Ballen in Perkolat eingeweicht, welches eine schwach saure Lösung darstellt. Alternativ können die Ballen jedoch auch in einer schwach alkalischen Lösung, wie beispielsweise Natronlauge, eingeweicht werden. Nach dem Einweichen werden die Ballen auf rund 40°C erwärmt, was beispielsweise dadurch bewerkstelligt werden kann, daß der Stroh-Kanal 46 (s. Fig. 8) durch Abwärme der Gas-Ottomotoren geheizt wird. Durch das Einweichen des Materials nach der Sattdampfbehandlung und vor der anaeroben bakteriellen Fermentation wird eine schwach aerobe Vorhydrolyse initiiert, durch die die anschließende anaerobe bakterielle Fermentation nochmals beschleunigt wird. Bei der Einweichung mit Perkolat sind die anaeroben Bakterien gleich am Ort frischen Materials, was ebenfalls vorteilhaft ist.

Es ist wichtig, festzuhalten, daß mit dem hier beschriebenen Verfahren zum Aufschluss von Stroh erreicht wird, daß das vorbehandelte Material in den Fermentern einen erheblichen Gasertrag bei moderaten Verweilzeiten erbringt, und dies, ohne zusätzliche Enzyme, Pilze oder Hefen zuzufügen. Der vorhandene natürliche Säuregehalt des Strohs (rund 3 bis 4 %) löst auch so die feste Cellulose auf und überführt sie in eine wäßrige Lösung (Autohydrolyse). Die biogenen Polymere werden durch die Einwirkung der organischen Säure chemisch und/oder durch Einfluß von Bakterien biochemisch in nieder-molekulare Verbindungen (Monosacharide, Aminosäuren, kurzkettige Peptide, langkettige Fettsäuren, Glyzerin) gespalten. Diese liegen am Ende der Phase in wassergelöster Form vor. Dies geschieht jedoch ohne daß zunächst Enzyme, Bakterien oder Hefen zugegeben werden müssen. Das ligninhaltige Material wird in dieser Ausführungsform allein der Autohydrolyse und der bakteriellen Hydrolyse überlassen.

Der hier im Detail beschriebene Aufschluss mit den genannten vier Verfahrensschritten ist äußerst effektiv und vorteilhaft, jedoch ist es nicht zwingend notwendig, daß sämtliche vier Schritte zur Anwendung kommen, und es können auch einfachere Verfahren mit weniger bzw. nur einer Auswahl der Schritte durchgeführt werden, die immer noch eine Vergärung von Stroh erlauben.

Hinsichtlich des vorzugsweisen chemischen Aufschlusses kann bereits dann ein brauchbarer Aufschluss des Strohs erhalten werden, wenn dieses vor der Einbringung in den Fermenter nur mit Festmist und/oder Gülle vermischt wird, weil durch den darin enthaltenen Harnstoff die Ligninstrukturen bereits aufweichen können. Es ist dabei sogar nicht unbedingt nötig, daß das Strohvor dem Einbringen in den Fermenter mit Gülle oder Festmist vermischt wird, sondern es kann bereits ausreichen, wenn das Stroh und Festmist in abwechselnden Lagen im Fermenter übereinandergeschichtet werden, gegebenenfalls mit dazwischen liegenden Schichten aus anderen, nicht lignifizierten NawaRos, wobei der Harnstoff der oben liegenden Festmistschichten mit dem Perkolat in die Schicht mit dem ligninhaltigen Material dringt und die flächigen Ligninstrukturen so zumindest teilweise auflöst. Dies stellt einen sehr einfachen Fall eines chemischen Aufschlusses dar.

### Bezugszeichenliste

- 10: Biomassekraftwerk
- 12: Grundabschnitt
- 14: Ausbauabschnitt
- 16: Fermenter
- 18: Fermentervorplatz
- 20: Fermentertor
- 22: Strom- und Wärmeerzeugungsanlage
- 24: Anliefer- und Verladebereich
- 26: Fermentervorplatz-Hallenabschnitt
- 28: Anliefer- und Verladebereichshallenabschnitt
- 30: Technikbrücke
- 31: Abgas-Abkühlraum
- 32: Folien-Gasspeicher
- 34: Perkolatumlauftank
- 36: Südlicher Technikraum
- 38: Nördlicher Technikraum
- 40: Lichtbänder
- 42: Anlieferbunker für Frischmasse
- 44: Förderband
- 46: Frischmassebunker
- 48: Bereitstellungsraum
- 50: Ballen-Anlieferungsraum
- 52: Aufschlußbereich
- 54: Zwischenlager
- 56: Rollenförderer
- 58: Strohkanal
- 60: Gärrestebunker
- 62: Schütttrog für Gärreste
- 64: Weiche für Gärreste
- 66: Förderband für Gärreste
- 68: Verladesilos für Gärreste
- 70: Dehydrierungseinrichtung
- 72: Strohballen
- 74: Unterseite des Strohballens 72
- 76: Oberseite des Strohballens 72
- 78: erster Satz von Löchern
- 80: zweiter Satz von Löchern
- 82: Einrichtung zur Sattdampfbehandlung
- 83: Deckel
- 84: Druckbehälter
- 85: Scharnier
- 86: Zentraler Gasverteilspeicher
- 87: Zuführeinrichtung
- 88: Motoraufstellraum
- 89: Behälter
- 90: Zuluft Motoraufstellräume
- 91: Schließmechanismus
- 92: Entlüftung Motoraufstellräume
- 93: Zufuhrleitung
- 94: Docking Station
- 95: Zuführungsventil
- 96: Lager
- 97: Auslaßleitung
- 98: Auslaß-Ventil
- 100: Kompressor
- 102: Sattdampf
- 103: Austretender Sattdampf
- 104: Einrichtung zur Sattdampfbehandlung
- 106: ligninhaltiger nachwachsender Rohstoff
- 108: druckfester Schieber
- 110: Trichter
- 112: Dampfreservoir
- 114: Heizung
- 116: Förderband
- 117: Slurry
- 118: Rohrverbindung
- 120: Leitung zum PUT
- 122: Einrichtung zur Sattdampfbehandlung
- 124: Spieß
- 126: innerer Hohlraum
- 128: Öffnung im Spieß 124
- 130: Einrichtung zur Sattdampfbehandlung
- 132: Kran
- 134: Laufkatze
- 136: Gestell
- 138: Greifer für oberen Ballen
- 140: Greifer für unteren Ballen

## Patentansprüche

1. Biogasanlage (10) zur Erzeugung von Biogas aus Stroh, umfassend:
eine Einrichtung zum mechanischen Aufschluss von Strohhalmen umfassend eine Vorrichtung zum mechanischen Zerkleinern von Strohhalmen durch Vermahlen, vorzugsweise Vermahlen mittels einer Hammermühle,
mindestens einen Fermenter für eine anaerobe bakterielle Vergärung der durch Vermahlen zerkleinerten Strohhalme im Nassfermentationsverfahren.

2. Biogasanlage nach Anspruch 1, wobei die Einrichtung zur Vermahlung der Strohhalme derart ist, dass die Vermahlung zu einer pulverförmigen Konsistenz erfolgt.

3. Biogasanlage nach Anspruch 1 oder 2, weiter aufweisend eine Einrichtung zum chemischen Aufschluss von Stroh, umfassend einen Behälter zum Einweichen des Strohs in Wasser, in einer Wasser-Säure-Lösung, in einer Wasser-Laugen-Lösung, in Perkolat oder in Gülle.

4. Biogasanlage nach einem der Ansprüche 1 bis 3, weiter aufweisend eine Einrichtung zum Häckseln der Strohhalme.

5. Verfahren zur Erzeugung von Biogas aus Stroh in einer Biogasanlage nach Anspruch 1, mit den folgenden Schritten:
Vorbehandlung der Strohhalme, um einen mechanischen Aufschluss desselben zu bewirken, wobei der mechanische Aufschluss der Strohhalme durch deren Vermahlung erfolgt, vorzugsweise Vermahlung mittels einer Hammermühle, Einbringen der durch Vermahlen zerkleinerten Strohhalme in einen Fermenter (16) zur Nassfermentierung, und
Erzeugen von Bedingungen im Fermenter (16), die eine anaerobe bakterielle Vergärung erlauben.

6. Verfahren nach Anspruch 5, bei dem zusätzlich ein chemischer Aufschluss des Strohs, durch Einweichen des Strohs, in Wasser, in einer Wasser-Säure-Lösung, in einer Wasser-Laugen-Lösung, in Perkolat oder in Gülle erfolgt.

7. Verfahren nach Anspruch 5 , bei dem die Vermahlung der Strohhalme zu einer pulverförmigen Konsistenz erfolgt.

8. Verfahren nach einem der Ansprüche 5 bis 7, bei dem die Strohhalme durch Häckseln vorzerkleinert werden.

## Claims

1. A biogas plant (10) for the production of biogas from straw, comprising:
a means for the mechanical decomposition of straw comprising a device for mechanically disintegrating of straw by grinding, preferably grinding with a hammer mill,
at least one fermenter for the anaerobic bacterial fermentation of the straw that had been disintegrated by grinding according to the wet fermentation method.

2. The biogas plant according to claim 1, wherein the device for grinding the straw is such that the grinding results in a powdery consistence.

3. The biogas plant according to claim 1 or 2, further comprising a device for the chemical break down of straw, comprising a container for soaking the straw in water, in a water-acid solution, in a water-lye solution, in percolate or in liquid manure.

4. The biogas plant according to any of claims 1 to 3, further comprising a device for chopping the straw.

5. A method for producing biogas from straw in a biogas plant according to claim 1, comprising the following steps:
pretreating the straw to effect a mechanical decomposition, wherein the mechanical decomposition of the straw is achieved by grinding it,
preferably grinding with a hammer mill,
introducing the straw disintegrated by grinding into a fermenter (16) for wet fermentation, and
producing conditions in the fermenter (16) which permit an anaerobic bacterial fermentation.

6. The method according to claim 5, wherein additionally a chemical decomposition of the straw is effected by soaking the straw in water, in a water-acid solution, in a water-lye solution, in percolate or in liquid manure.

7. The method according to claim 5, wherein the grinding of the straw is to achieve a powdery texture.

8. The method according to any of claims 5 to 7, wherein the straw is pre-cut by chopping it.

## Revendications

1. Installation de biogaz (10) destinée à la production de biogaz à partir de paille, comportant:
un dispositif de réduction mécanique de paillettes comportant un dispositif de trituration mécanique des paillettes par broyage, de préférence par broyage à l'aide d'un broyeur à marteaux,
au moins un fermenteur destiné à une fermentation bactérienne anaérobie des paillettes triturées dans le processus de fermentation humide.

2. Installation de biogaz selon la revendication 1, dans laquelle le dispositif de trituration des paillettes est tel qu'ait lieu le broyage pour obtenir une consistance pulvérulente.

3. Installation de biogaz selon la revendication 1 ou 2, présentant par ailleurs un dispositif de réduction chimique de la paille, comportant un récipient pour tremper la paille dans de l'eau, dans une solution eau-acide, dans une solution eau-alcali, dans du percolât ou dans du lisier.

4. Installation de biogaz selon l'une des revendications 1 à 3, présentant par ailleurs un dispositif de hachage des paillettes.

5. Procédé de production de biogaz à partir de paille dans une installation de biogaz selon la revendication 1, aux étapes suivantes consistant à:
prétraiter les paillettes pour provoquer une réduction mécanique de ces dernières, où la réduction mécanique des paillettes a lieu par broyage de ces dernières, de préférence par broyage au moyen d'un broyeur à marteaux,
introduire les paillettes triturées par broyage dans un fermenteur (16) pour une fermentation humide, et
créer dans le fermenteur (16) des conditions qui permettent une fermentation bactérienne anaérobie.

6. Procédé selon la revendication 5, dans lequel a lieu en outre une réduction chimique de la paille, par trempage de la paille dans de l'eau, dans une solution eau-acide, dans une solution eau-alcali, dans du percolât ou dans du lisier.

7. Procédé selon la revendication 5, dans lequel a lieu le broyage des paillettes de manière à obtenir une consistance pulvérulente.

8. Procédé selon l'une des revendications 5 à 7, dans lequel les paillettes sont triturées par hachage.
